# EUROPEAN PATENT APPLICATION

(11) **EP 4 843 665 A1**
(43) Date of publication of application: **25.11.2026**
(21) Application number: 24952789.6
(22) Date of filing: 31.12.2024
(51) Int. Cl.: B01J 29/70, C01B 39/04, C01B 39/48

(54) **ZSM-23 MOLECULAR SIEVE, AND PREPARATION METHOD THEREFOR AND USE THEREOF**

(30) Priority: 30.08.2024 CN 202411211094
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: CHEN, Yujing, Dalian, Liaoning 116045 (CN); YU, Zhengmin, Dalian, Liaoning 116045 (CN); YU, Tao, Dalian, Liaoning 116045 (CN); FAN, Hongfei, Dalian, Liaoning 116045 (CN); SUN, Xiaoyan, Dalian, Liaoning 116045 (CN)
(74) Representative: Hoffmann Eitle
(86) International application number: PCT/CN2024/144199
(87) International publication number: WO 2026/045064

(57) **Abstract**

The present invention relates to the technical field of molecular sieve preparation. Disclosed are a ZSM-23 molecular sieve, and a preparation method therefor and the use thereof. The ratio of the total Brønsted acidity determined via 2,6-dimethylpyridine adsorption FTIR to the total Brønsted acidity determined via pyridine adsorption FTIR of the ZSM-23 molecular sieve is 65-99 : 100. Moreover, in the 2,6-dimethylpyridine infrared B acid, the ratio of the Bronsted acidity at a desorption temperature below 250°C to the total Brønsted acidity determined via 2,6-dimethylpyridine adsorption FTIR is 64-95 : 100. The Bronsted acidic sites of the ZSM-23 molecular sieve of the present invention are predominantly distributed at orifices and on the external surface, and with weak Brønsted acidic sites being dominant. The molecular sieve exhibits excellent catalytic reaction performance mainly based on the pore-mouth adsorption mechanism.

## Description

### CROSS-REFERENCE TO RELATED APPLICATIONS

The present application claims the benefit of China patent application No. "202411211094.7", filed on August 30, 2024, the content of which is specifically and entirely incorporated herein by reference.

### TECHNICAL FIELD

The present invention relates to the technical field of preparation of molecular sieve, in particular to a ZSM-23 molecular sieve, a preparation method therefor and use thereof.

### BACKGROUND ART

ZSM-23 molecular sieve is a microporous molecular sieve featuring a high silica-to-alumina ratio. Benefiting from its distinctive pore and channel architecture, it has found extensive applications in separation, adsorption and catalysis. Within petrochemical industries, ZSM-23 molecular sieve delivers outstanding catalytic performance in reactions including hydrocracking of long-chain alkanes and olefins as well as alkane isomerization, endowing it with promising application prospects and considerable market potential.

Patent application CN109694082A discloses a preparation method of ZSM-23 molecular sieve. The preparation method comprises the following steps: (1) mixing a silicon source, an aluminum source, an alkali source and an organic template agent a in water uniformly to obtain a gel a; (2) mixing a silicon source, an aluminum source, an alkali source, an organic template agent b and a seeds in water uniformly to obtain a gel, then transferring into a crystallization kettle after mixing uniformly, and crystallizing at the temperature of 100-200°C for 1-48 hours; (3) after the crystallizing process of step (2) is finished, recovering the organic template agent b to obtain a first-stage microcrystalline solution; and (4) adding the gel a to the first-stage microcrystalline solution obtained in step (3); after the crystallization process is finished, recovering the organic template agent a to obtain a molecular sieve slurry; and separating mother liquor of the slurry, washing and drying to obtain raw ZSM-23 molecular sieve powder. The ZSM-23 molecular sieve synthesized in the patent application fails to regulate and control the distribution of acid sites, when the ZSM-23 molecular sieve is used in the shape-selective catalytic reaction process such as hydroisomerization, toluene disproportionation and xylene isomerization. The acidic sites located in the pores and channels of ZSM-23 molecular sieve exhibit poor accessibility and low utilization efficiency in most reactions due to steric hindrance and diffusion limitations.

Patent application CN109516471A discloses a synthesizing method of a surface aluminum enriched type ZSM-23 molecular sieve. In the initial synthesis stage, a molecular sieve gel is first prepared and pre-crystallized, which generates abundant molecular sieve crystal nuclei with a high silicon-aluminum ratio. Subsequently, a template-free gel with a low silicon-aluminum ratio is introduced. After further crystallization, an Al-enriched ZSM-23 molecular sieve with a surface silicon-aluminum ratio ranging from 10 to 80 is obtained. By means of the method, the surface aluminum enriched type ZSM-23 molecular sieve can be synthesized in a short time with a lower dosage of template agent. The number of acidic centers at orifices and on the external surface is increased, and the crystal size is reduced. However, the method does not precisely regulate and control the aluminum atoms at orifices and on the external surface. The weak Brønsted acidity at these positions is relatively low, whereas the concentration of strong Brønsted acidic sites is considerably high. While the ZSM-23 molecular sieve favors the shape-selective catalytic reaction at pore mouths, it also facilitates the occurrence of side reactions.

### SUMMARY OF THE INVENTION

The present invention aims to overcome the defects in the prior art that the distribution of acidic sites in the ZSM-23 molecular sieve is relatively disperse, the Brønsted acidity at orifices and on the external surface of the ZSM-23 molecular sieve is relatively low, such that the ZSM-23 molecular sieve exhibits a poor reaction performance mainly based on the orifice adsorption mechanism, and provides a ZSM-23 molecular sieve, a preparation method therefor and use thereof. The Brønsted acidic sites of the ZSM-23 molecular sieve of the present invention are predominantly distributed at orifices and on the external surface, and with weak Brønsted acidic sites being dominant, the ZSM-23 molecular sieve exhibits excellent catalytic reaction performance mainly based on the pore-mouth adsorption mechanism.

In order to achieve the above objects, the first aspect of the present invention provides a ZSM-23 molecular sieve, the ZSM-23 molecular sieve possesses a ratio of total Brønsted acidity determined via 2,6-dimethylpyridine adsorption FTIR to total Brønsted acidity determined via pyridine adsorption FTIR being (65-99): 100, preferably (70-97): 100, and more preferably (74-95): 100; in the Brønsted acidity determined via 2,6-dimethylpyridine adsorption FTIR, the ratio of Brønsted acidity at a desorption temperature below 250°C to the total Brønsted acidity determined via 2,6-dimethylpyridine adsorption FTIR is (64-95): 100, preferably (67-93):100, and more preferably (70-91): 100.

The second aspect of the present invention provides a method for preparing ZSM-23 molecular sieve, the method comprises the following steps:
(1) Crystallizing a mixture of silicon source, template agent a and water, and then separating the solid-phase material from the resultant product;
(2) Mixing the above solid-phase material with aliphatic amine and inorganic base for a primary reaction, subsequently adding ZSM-23 seeds and proceeding with a secondary reaction;
(3) Blending the mixed material obtained in step (2) with an aluminum source and a template agent b to obtain a mixture, followed by crystallization, filtration, washing, drying and calcination in sequence.

The third aspect of the present invention provides a ZSM-23 molecular sieve prepared with the aforementioned method.

The fourth aspect of the present invention provides a hydrogenation catalyst comprising a support and an active component attached on the support, wherein the support is the aforementioned ZSM-23 molecular sieve.

The fifth aspect of the present invention relates to the application of the aforementioned hydrogenation catalyst in the long-chain alkane hydrocracking, olefin hydrocracking or alkane isomerization reactions.

For the ZSM-23 molecular sieve of the present invention, Brønsted acidic sites are mainly located at orifices and the external surface and dominated by weak Brønsted acidic sites. This molecular sieve delivers high catalytic activity and superior performance for reactions mainly based on the pore-mouth adsorption mechanism.

In the preparation method of ZSM-23 molecular sieve according to the present invention, a mixture containing metastable pure silicon ten-membered ring molecular sieve nanocrystals is initially prepared by using a silicon source, a template agent, an aliphatic amine and an inorganic base. The resulting mixture is then blended with an aluminum source and a template agent for crystallization. Herein, a chelate formed by coupling of the aluminum source to the template agent is capable of combining with the hydroxyl groups on the surface of the metastable pure-silicon ten-membered ring molecular sieve nanocrystals, and embedded into the skeletal structure and grown into structurally stable ZSM-23 molecular sieve with a surface enriched in weak Brønsted acidic sites during the crystallization process. The template agent used in the method is inexpensive and requires a low dosage. This technique enables precise regulation of Al sites in ZSM-23 molecular sieve, facilitates the enrichment of weak Brønsted acidic sites at orifices and on the external surface, and remarkably improves the reaction performance of ZSM-23 molecular sieve governed by the pore-mouth adsorption mechanism.

### BRIEF DESCRIPTION OF THE DRAWINGS

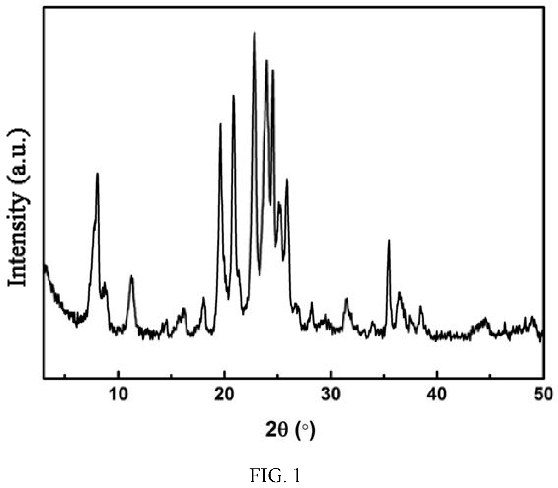

FIG. 1 illustrates an XRD spectrogram of the ZSM-23 molecular sieve prepared in Example 1 of the present invention.

### DESCRIPTION OF THE PREFERRED EMBODIMENT

The specific embodiments of the present invention are described in details below. It should be understood that the specific embodiments described herein merely serve to illustrate and explain the present invention, instead of imposing limitations thereon.

In the present invention, the term "the total Brønsted acidity determined via pyridine adsorption FTIR" serves to express the total Brønsted acidity in the ZSM-23 molecular sieve, including the Brønsted acidic sites on the external surface, at orifices and in channel of the molecular sieve. The specific process is as follows: ZSM-23 molecular sieve sample was prepared into a self-supporting wafer with a loading of 5-6 mg/cm²which is placed in an in-situ pool, treated in vacuum at a temperature of 400°C for 4 hours and then cooled to 50°C for a spectrum acquisition; after pyridine adsorption for 10min, the sample was heated to 150°C and subjected to desorption for 1h; upon cooling to room temperature, a spectrogram was collected, and the total Brønsted acidity via pyridine adsorption FTIR was obtained by calculation, wherein the Brønsted acidity was calculated according to the Lambert-Beer law, and the area of 1540 cm⁻¹ absorption peak was used for calculating the Brønsted acidity.

In the present invention, the term "the total Brønsted acidity determined via 2,6-dimethylpyridine adsorption FTIR" serves to indicatthe Brønsted acidic sites distributed at orifices and on the external surface of the ZSM-23 molecular sieve. The specific process is as follows: the sample was fabricated into a self-supporting wafer with a loading of 5-6 mg/cm² and placed in an in-situ pool, treated in vacuum at a temperature of 400°C for 4 hours and then cooled to 50°C for a spectrum acquisition. After adsorption of 2,6-dimethylpyridine for 10min, the sample was heated to 150°C and subjected to desorption for 1h, cooled to room temperature and a spectrum was collected. The total Brønsted acidity was calculated via infrared spectrum using 2,6-dimethylpyridine as the probe molecule. The sample was further heated to 250°C for another 1h of desorption. After cooling to room temperature, a spectrogram was collected again to determine the weak Brønsted acidity at a desorption temperature below 250°C wherein the Brønsted acidity was calculated according to the Lambert-Beer law, using the integrated areas of the absorption bands at 1630cm⁻¹ and 1650cm⁻¹.

In the present invention, the term "Brønsted acidity at a desorption temperature below 250°C" serves to express the weak Brønsted acidity. The term "Brønsted acidity at a desorption temperature below 250°C in the 2,6-dimethylpyridine adsorption FTIR Brønsted acidity" refers to the weak Brønsted acidity distributed at orifices and on the external surface of ZSM-23 molecular sieve.

In the invention, the bulk-phase SiO₂/Al₂O₃ molar ratio was analyzed through the X-ray fluorescence spectroscopy (XRF). A ZSX100e X-ray fluorescence spectrometer was used with the following test parameters: Kα spectral line, LiF1 analyzing crystal, Rh target, SC scintillation detector, 20 s counting time, and vacuum optical path atmosphere.

In the present invention, the SiO₂/Al₂O₃ molar ratio on the external surface was measured by X-ray photoelectron spectroscopy (XPS). The elemental composition and state on the catalyst surface were determined by using the Multilab2000 type electron spectrometer manufactured by Thermofisher Inc. in the United States of America (USA), wherein an excitation source was Mg Kα, and the cathode voltage and current were 13kV and 20mA, respectively. The electron binding energy was calibrated with C1s(284.6eV).

In the ZSM-23 molecular sieve according to the present invention, the Brønsted acidic sites are mainly distributed at pore mouths and on the external surface. Specifically, the ratio of the total Brønsted acidity determined via 2,6-dimethylpyridine adsorption FTIR to the total Brønsted acidity determined via pyridine adsorption FTIR is (65-99): 100, preferably (70-97): 100, and more preferably (74-95): 100. That is, 65-99% (preferably 70-97%, more preferably 74-95%) of the Brønsted acidic sites in the ZSM-23 molecular sieve are distributed at orifices and on the external surface.

In the ZSM-23 molecular sieve according to the present invention, the Brønsted acidic sites at orifices and on the external surface are mainly weak Brønsted acidic sites. Particularly, in the Brønsted acidity determined via 2,6-dimethylpyridine adsorption FTIR, the ratio of Brønsted acidity at a desorption temperature below 250°C to the total Brønsted acidity determined via 2,6-dimethylpyridine adsorption FTIR is (64-95): 100, preferably (67-93):100, and more preferably (70-91): 100. That is, 64-95% (preferably 67-93%, more preferably 70-91%) of the Brønsted acidic sites at orifices and on the external surface of the ZSM-23 molecular sieve are weak Brønsted acidic sites.

In the ZSM-23 molecular sieve according to the present invention, the total Brønsted acidity determined via pyridine adsorption FTIR in the ZSM-23 molecular sieve may be within the range of 0.1-0.37mmol/g, preferably within the range of 0.12-0.36mmol/g, further preferably within the range of 0.13-0.35mmol/g, and more preferably within the range of 0.15-0.33mmol/g. When the total Brønsted acidity determined via pyridine adsorption FTIR in the ZSM-23 molecular sieve falls into the aforementioned preferred range, the ZSM-23 molecular sieve exhibits a higher catalytic activity.

In the ZSM-23 molecular sieve according to the present invention, the total Brønsted acidity determined via 2,6-dimethylpyridine adsorption FTIR in the ZSM-23 molecular sieve may be within the range of 0.07-0.35mmol/g, preferably within the range of 0.09-0.33mmol/g, and more preferably within the range of 0.11-0.30mmol/g. When the total Brønsted acidity determined via 2,6-dimethylpyridine adsorption FTIR in the ZSM-23 molecular sieve falls into the aforementioned preferred range, the ZSM-23 molecular sieve not only possesses a high catalytic activity, but also exhibits excellent catalytic reaction performance dominated by the pore-mouth adsorption mechanism.

In the ZSM-23 molecular sieve according to the present invention, the Brønsted acidity at a desorption temperature below 250°C in the Brønsted acidity determined via 2,6-dimethylpyridine adsorption FTIR of the ZSM-23 molecular sieve may be within the range of 0.06-0.31mmol/g, preferably within the range of 0.07-0.30mmol/g, further preferably within the range of 0.09-0.29mmol/g, and more preferably within the range of 0.10-0.26mmol/g. When the Brønsted acidity at a desorption temperature below 250°C in the Brønsted acidity determined via 2,6-dimethylpyridine adsorption FTIR of the ZSM-23 molecular sieve falls into the aforementioned preferred range, the ZSM-23 molecular sieve not only possesses a high catalytic activity, but also exhibits excellent catalytic reaction performance mainly based on the pore-mouth adsorption mechanism.

In the ZSM-23 molecular sieve according to the present invention, the Al sites are mainly distributed on the external surface of the molecular sieve, thereby facilitating the enrichment of weak Brønsted acidic sites at orifices and on the external surface of the molecular sieve. Therefore, the bulk-phase SiO₂/Al₂O₃ molar ratio of the ZSM-23 molecular sieve according to the present invention is apparently higher than the external surface SiO₂/Al₂O₃ molar ratio. In a case of preferably, the ZSM-23 molecular sieve possesses the bulk-phase SiO₂/Al₂O₃ molar ratio higher than the external surface SiO₂/Al₂O₃ molar ratio by an amount of 40-400, preferably an amount of 45-350, and more preferably by an amount of 50-320.

In the ZSM-23 molecular sieve according to the present invention, the bulk-phase SiO₂/Al₂O₃ molar ratio of the ZSM-23 molecular sieve may be within the range of 70-500, preferably within the range of 90-400, and more preferably within the range of 120-300.

In the ZSM-23 molecular sieve according to the present invention, the external surface SiO₂/Al₂O₃ molar ratio of the ZSM-23 molecular sieve may be within the range of 40-120, preferably within the range of 45-90, and more preferably within the range of 50-80.

In some embodiments, the bulk-phase SiO₂/Al₂O₃ molar ratio of the ZSM-23 molecular sieve is within the range of 70-500 and the external surface SiO₂/Al₂O₃ molar ratio is within the range of 40-120, and the bulk-phase SiO₂/Al₂O₃ molar ratio is higher than the external surface SiO₂/Al₂O₃ molar ratio by an amount of 40-400. The total Brønsted acidity determined via pyridine adsorption FTIR in the ZSM-23 molecular sieve is within the range of 0.1-0.37mmol/g, the total Brønsted acidity determined via 2,6-dimethylpyridine adsorption FTIR is within the range of 0.07-0.35mmol/g, and the ratio of the total Brønsted acidity determined via 2,6-dimethylpyridine adsorption FTIR to the total Brønsted acidity determined via pyridine adsorption FTIR is (65-99): 100; and in the Brønsted acidity determined via 2,6-dimethylpyridine adsorption FTIR, the ratio of the Brønsted acidity at a desorption temperature below 250°C to the total Brønsted acidity determined via 2,6-dimethylpyridine adsorption FTIR is (64-95): 100. According to the embodiment, the ZSM-23 molecular sieve not only displays a high catalytic activity, but also exhibits desirable catalytic reaction performance mainly based on the pore-mouth adsorption mechanism.

In other embodiments, the bulk-phase SiO₂/Al₂O₃ molar ratio of the ZSM-23 molecular sieve is within the range of 90-400 and the external surface SiO₂/Al₂O₃ molar ratio is within the range of 45-90, and the bulk-phase SiO₂/Al₂O₃ molar ratio is higher than the external surface SiO₂/Al₂O₃ molar ratio by an amount of 45-350. The total Brønsted acidity determined via pyridine adsorption FTIR in the ZSM-23 molecular sieve is within the range of 0.1-0.37mmol/g, the total Brønsted acidity determined via 2,6-dimethylpyridine adsorption FTIR is within the range of 0.07-0.35mmol/g, and the ratio of the total Brønsted acidity determined via 2,6-dimethylpyridine adsorption FTIR to the total Brønsted acidity determined via pyridine adsorption FTIR is (70-97): 100; and in the Brønsted acidity determined via 2,6-dimethylpyridine adsorption FTIR, the ratio of the Brønsted acidity at a desorption temperature below 250°C to the total Brønsted acidity determined via 2,6-dimethylpyridine adsorption FTIR is (67-93): 100. According to the embodiment, the catalytic activity of the ZSM-23 molecular sieve is further enhanced, and the ZSM-23 molecular sieve exhibits better catalytic reaction performance mainly based on the pore-mouth adsorption mechanism.

In other preferred embodiments, the bulk-phase SiO₂/Al₂O₃ molar ratio of the ZSM-23 molecular sieve is within the range of 90-400 and the external surface SiO₂/Al₂O₃ molar ratio is within the range of 45-90, and the bulk-phase SiO₂/Al₂O₃ molar ratio is higher than the external surface SiO₂/Al₂O₃ molar ratio by an amount of 45-350. The total Brønsted acidity determined via pyridine adsorption FTIR in the ZSM-23 molecular sieve is within the range of 0.12-0.36mmol/g, the total Brønsted acidity determined via 2,6-dimethylpyridine adsorption FTIR is within the range of 0.09-0.33mmol/g, and the ratio of the total Brønsted acidity determined via 2,6-dimethylpyridine adsorption FTIR to the total Brønsted acidity determined via pyridine adsorption FTIR is (70-97): 100; and in the Brønsted acidity determined via 2,6-dimethylpyridine adsorption FTIR, the ratio of the Brønsted acidity at a desorption temperature below 250°C to the total Brønsted acidity determined via 2,6-dimethylpyridine adsorption FTIR is (67-93): 100. According to the embodiment, the ZSM-23 molecular sieve demonstrates a better catalytic activity, and the catalytic reaction performance dominated by the pore-mouth adsorption mechanism is further improved.

In other further preferred embodiments, the bulk-phase SiO₂/Al₂O₃ molar ratio of the ZSM-23 molecular sieve is within the range of 120-300 and the external surface SiO₂/Al₂O₃ molar ratio is within the range of 50-80, and the bulk-phase SiO₂/Al₂O₃ molar ratio is higher than the external surface SiO₂/Al₂O₃ molar ratio by an amount of 50-320. The total Brønsted acidity determined via pyridine adsorption FTIR in the ZSM-23 molecular sieve is within the range of 0.12-0.36mmol/g, the total Brønsted acidity determined via 2,6-dimethylpyridine adsorption FTIR is within the range of 0.09-0.33mmol/g, and the ratio of the total Brønsted acidity determined via 2,6-dimethylpyridine adsorption FTIR to the total Brønsted acidity determined via pyridine adsorption FTIR is (74-95): 100; and in the Brønsted acidity determined via 2,6-dimethylpyridine adsorption FTIR, the Brønsted acidity at a desorption temperature below 250°C is within the range of 0.10-0.31mmol/g, and the ratio of the Brønsted acidity at a desorption temperature below 250°C to the total Brønsted acidity determined via 2,6-dimethylpyridine adsorption FTIR is (70-91): 100. According to the embodiment, the ZSM-23 molecular sieve not only exhibits excellent catalytic activity, but also can obtain the optimum catalytic reaction performance dominated by the pore-mouth adsorption mechanism.

The method for preparing the ZSM-23 molecular sieve according to the present invention comprises the following steps:
(1) Crystallizing a mixture of silicon source, template agent a and water, and then separating the solid-phase material from the resultant product;
(2) Mixing the above solid-phase material with aliphatic amine and inorganic base for a primary reaction, subsequently adding ZSM-23 seeds and proceeding with a secondary reaction;
(3) Blending the mixed material obtained in step (2) with an aluminum source and a template agent b to obtain a mixture, followed by crystallization, filtration, washing, drying and calcination in sequence.

According to the preparation method of the ZSM-23 molecular sieve in the present invention, the precise regulation and control of the Al sites of the ZSM-23 molecular sieve can be realized, so that the Al sites are mainly distributed on the external surface of the ZSM-23 molecular sieve. It is conducive to the enrichment of weak Brønsted acidic sites at orifices and on the external surface of the molecular sieve. As a result, the obtained ZSM-23 molecular sieve not only demonstrates a high catalytic activity, but also possesses obviously improved catalytic reaction performance dominated by the pore-mouth adsorption mechanism.

The molar ratio of water, said template a, and said silicon source calculated in terms of SiO₂ in step (1) may be (20-80): (0.1-1): 1, preferably (30-70): (0.15-0.8): 1.

In step (1), the silicon source may be a silicon source conventionally employed in the art. In a specific embodiment, the silicon source may be selected from, but not limited to, one or a random combination of at least two of white carbon black, silica sol, sodium silicate, fumed silica and tetraethyl orthosilicate. In a preferred embodiment, the silicon source is white carbon black and/or fumed silica.

In the step (1), the template agent a refers to a template agent capable of directing the production of secondary structural units (e.g., a ten-membered ring structure) of ZSM-23 in pure-silica state. The specific material types are not particularly limited in the present invention, and the conventional template agents in the field may be selected. In a specific embodiment, the template agent a may be selected from, but not limited to, one or a random combination of at least two of hexamethylene diamine, n-hexylamine, ethanol, tetrapropyl ammonium hydroxide, tetrapropyl ammonium bromide and triethylamine. In a more preferred embodiment, the template agent a is hexamethylene diamine and/or ethanol.

In step (1), the crystallization reaction conditions may comprise: a temperature within the range of 120-220°C, preferably within the range of 140-200°C, and a duration of 8-48 hours, preferably 12-30 hours.

In step (1), the crystallization reaction is preferably carried out in a reaction kettle equipped with a polytetrafluoroethylene lining.

In step (1), the process of separating a solid-phase material from the materials following said reaction is solid-liquid separation. The solid-liquid separation mode can be selected from, but not limited to, any one of gravity settling, filtration separation and centrifugal separation, preferably centrifugal separation.

In step (2), the liquid-solid ratio of the aliphatic amine to the solid-phase material in step (2) may be within the range of 0.3-3 mL/g, preferably within the range of 0.5-2 mL/g.

In step (2), the aliphatic amine is added to promote the regular arrangement of crystal, thereby facilitating the formation of metastable pure silicon ten-membered ring molecular sieve nanocrystals. The aliphatic amine may be C₁₂-C₁₈ aliphatic amine. In a specific embodiment, the aliphatic amine is selected from, but not limited to, at least one of oleylamine (i.e., 9-octadecenylamine), octadecylamine and dodecylamine. In a most preferred embodiment, the aliphatic amine is oleylamine.

The inorganic base in step (2) is used in the form of an alkali solution. The concentration of the alkali solution may be within the range of 0.003-0.015mol/L, preferably within the range of 0.005-0.01 mol/L.

In step (2), the liquid-solid ratio of the alkali solution to the solid-phase material may be within the range of 2-15 mL/g, preferably within the range of 4-10 mL/g.

In the present invention, the inorganic base may be selected from, but not limited to, at least one selected from the group of sodium hydroxide, potassium hydroxide and aqueous ammonia.

The temperature of the secondary reaction in step (2) is higher than that of the primary reaction. In a case of preferably, the temperature of the secondary reaction is 30-90°C, preferably 40-80°C higher than the temperature of the primary reaction.

In step (2), the conditions of the primary reaction may comprise: a temperature within the range of 20-40°C, preferably within the range of 25-30°C, and a duration of 3-15 hours, preferably 6-12 hours. The primary reaction is preferably carried out under the stirring conditions, and the stirring speed may be 100-300 rpm.

In step (2), the conditions of the secondary reaction may comprise: a temperature within the range of 60-120°C, preferably within the range of 80-100°C, and a duration of 6-30 hours, preferably 12-24 hours. The secondary reaction is preferably carried out under the stirring conditions, and the stirring speed may be 100-300 rpm.

In step (2), the dosage of the ZSM-23 seeds is not particularly limited, and routine seed dosages commonly used in the field may be adopted. In a specific embodiment, the dosage of said ZSM-23 seeds in step (2) is 0.1-8 parts by weight, preferably 0.5-5 parts by weight, relative to 100 parts by weight of the solid-phase material.

In step (2), the specific operation process of blending the solid-phase material with aliphatic amine and inorganic base are preferably as follows: blending the solid-phase material obtained in step (1) with the aliphatic amine, and then adding the alkali solution into the mixed material.

In step (2), the specific operation process of adding the ZSM-23 seeds is preferably as follows: ZSM-23 seeds are dispersed in deionized water to form a seed liquid, the seed liquid is then added. The liquid-solid ratio of the deionized water to the ZSM-23 seeds may be 5-70 mL/g, preferably 10-60 mL/g.

According to the preparation method of ZSM-23 molecular sieve in the present invention, the molar ratio of the dosage of said template b, the aluminum source calculated in terms of Al₂O₃, and the mixed material calculated in terms of SiO₂ in step (3) may be (0.01-0.1): (0.002-0.015): 1, preferably (0.02-0.08): (0.005-0.01): 1.

In step (3), the specific operation process of blending the aluminum source, the template agent b and the mixed materials is preferably as follows: preparing an aqueous solution containing an aluminum source and a template agent b, and then blending the aqueous solution with the mixed material to obtain a gel. In the aqueous solution containing an aluminum source and a template agent b, the molar ratio of water to the aluminum source (calculated in terms of Al₂O₃) may be within the range of 300-3,000, preferably within the range of 400-2,000.

In the present invention, the template agent b is a template agent capable of directing the formation of ZSM-23 molecular sieve, and the specific species of said template agent is not particularly limited in the invention, and a conventional template agent in the field can be selected. In a specific embodiment, the template b may be selected from, but not limited to, one or a random combination of at least two of pyrrolidine, isopropylamine, N,N-dimethylformamide, dimethylamine and ethylenediamine. In a most preferred embodiment, the template agent b is pyrrolidine.

In the present invention, the aluminum source may be any aluminum source commonly used in the field. In a specific embodiment, the aluminum source may be selected from, but not limited to, one or a random combination of at least two of aluminum sulfate, aluminum isopropoxide, sodium aluminate and aluminum hydroxide. In a most preferred embodiment, the aluminum source is aluminum sulfate.

The crystallizing conditions in step (3) may comprise: a temperature within the range of 180-220°C, and a duration of 24-72 hours.

The drying conditions in step (3) may comprise: a temperature within the range of 80-120°C, and a duration of 6-12 hours.

The calcining conditions in step (3) may comprise: a temperature within the range of 540-560°C, and a time of 3-8 hours.

The ZSM-23 molecular sieve prepared with the method according to the present invention enables precise regulation and control of Al sites, primarily distributing them on the external surface. It facilitates the enrichment of weak Brønsted acidic sites at the pore mouths and on the external surface of the molecular sieve, which not only endows the ZSM-23 molecular sieve with outstanding catalytic reaction performance dominated by the pore-mouth adsorption mechanism.

The present invention also provides a ZSM-23 molecular sieve prepared with the aforementioned method. The Brønsted acidic sites of the ZSM-23 molecular sieve are mainly distributed at orifices and on the external surface, dominated by weak Brønsted acidic sites. The ZSM-23 molecular sieve exhibits excellent catalytic reaction performance mainly based on the pore-mouth adsorption mechanism.

The hydrogenation catalyst in the present invention comprises a support and an active component attached on the support, wherein the support is the aforementioned ZSM-23 molecular sieve.

In the hydrogenation catalyst, the content of the active component may be within the range of 0.1-5 wt.%, preferably within the range of 0.2-2 wt.%. The active component may be a metal active component commonly used in the field. In a preferred embodiment, the active component is a precious metal component (e.g., Pt).

The invention further provides a use of the aforementioned hydrogenation catalyst in the long-chain alkane hydrocracking, olefin hydrocracking or alkane isomerization reactions.

In practical application, the specific distribution characteristics of Brønsted acidic sites in the ZSM-23 molecular sieve support enable an excellent catalytic performance mainly based on the pore-mouth adsorption mechanism.

The ZSM-23 molecular sieve, a preparation method therefor and use thereof according to the present invention are further described below with reference to examples. The examples are implemented on the premise of the technical scheme of the present invention, and provide the detailed embodiments and specific operation processes, but the protection scopes of the present invention are not limited to the following examples.

Unless otherwise specified, the experimental methods in the following examples were conventional methods in the field. Unless otherwise specified, the experimental materials used in the following examples were commercially available.

### Example 1

4.68g of hexamethylene diamine was dissolving in 54g of deionized water, followed by the addition of 6g of fumed silica. The mixture was stirred at room temperature (25°C) for 1 hour, and then transferred to a 100mL autoclave equipped with a polytetrafluoroethylene liner. The crystallization reaction was performed at 150°C for 24 hours. After rapid quenching and centrifugal separation, 6.43g of a solid-phase material was obtained. 10mL of oleylamine was mixed with the aforementioned solid-phase material, and the mixture was thoroughly homogenized using an oscillator, followed by stirring at room temperature for 0.5 hour at a stirring speed of 200 rpm. Then 42mL of NaOH solution with a concentration of 0.008mol/L was subsequently added into the mixed liquor, and the primary reaction was carried out at a temperature of 25°C under stirring with a stirring speed of 200rpm for 6 hours to obtain Mixture A. Subsequently, 0.18g of ZSM-23 seeds were dispersed in deionized water to prepare 10mL of ZSM-23 seed suspension, which was then introduced into the Mixture A. The secondary reaction was performed at 80°C under stirring with a stirring speed of 300rpm for 16 hours, yielding Mixture B.

0.67g of Al₂(SO₄)₃·18H₂O and 0.40g of pyrrolidine were added into 20mL of deionized water in sequence to obtain a clear solution C. The solution C was incorporated into the Mixture B and then uniformly blended. The final mixture was transferred into a 150mL autoclave equipped with a polytetrafluoroethylene liner and subjected to crystallization at 180°C for 48 hours. After crystallization, the product was filtered, washed, dried at 100°C for 12 hours, and calcined at 550°C for 4 hours. A ZSM-23 molecular sieve denoted as Z-1 was obtained and its XRD spectrogram was shown in FIG. 1.

According to the characterization results of pyridine adsorption FTIR and 2,6-dimethylpyridine adsorption FTIR spectra, the total Brønsted acidity determined via pyridine adsorption FTIR of the molecular sieve Z-1 was 0.184mmol/g, and the total Brønsted acidity determined via 2,6-dimethylpyridine adsorption FTIR was 0.164mmol/g, with their ratio of 89.1: 100. In the Brønsted acidity determined via 2,6-dimethylpyridine adsorption FTIR, the Brønsted acidity at a desorption temperature below 250°C (i.e., the week Brønsted acidity) was 0.149mmol/g. The ratio of the Brønsted acidity at a desorption temperature below 250°C to the total Brønsted acidity determined via 2,6-dimethylpyridine adsorption FTIR was 90.8: 100.

The bulk-phase SiO₂/Al₂O₃ molar ratio of the molecular sieve Z-1 was 97, and the external surface SiO₂/Al₂O₃ molar ratio was 46.

### Example 2

17.43g of hexamethylene diamine was dissolving in 216g of deionized water, followed by the addition of 18g of fumed silica. The mixture was stirred at room temperature (25°C) for 1 hour, and then transferred to a 300mL autoclave equipped with a polytetrafluoroethylene liner. The crystallization reaction was performed at 200°C for 12 hours. After rapid quenching and centrifugal separation, 18.97g of a solid-phase material was obtained.

37mL of oleylamine was mixed with the aforementioned solid-phase material, and the mixture was thoroughly homogenized using an oscillator, followed by stirring at room temperature for 0.5 hour at a stirring speed of 150 rpm. Then 76mL of NaOH solution with a concentration of 0.01mol/L was subsequently added into the mixed liquor, and the primary reaction was carried out at a temperature of 25°C under stirring with a stirring speed of 150rpm for 12 hours to obtain Mixture A. Subsequently, 0.9g of ZSM-23 seeds were dispersed in deionized water to prepare 30mL of ZSM-23 seed suspension, which was then introduced into the Mixture A. The secondary reaction was performed at 100°C under stirring with a stirring speed of 300rpm for 20 hours, yielding Mixture B.

0.67g of Al₂(SO₄)₃·18H₂O and 0.85g of pyrrolidine were added into 20mL of deionized water in sequence to obtain a clear solution C. The solution C was incorporated into the Mixture B and then uniformly blended. The final mixture was transferred into a 200mL autoclave equipped with a polytetrafluoroethylene liner and subjected to crystallization at 200°C for 36 hours. After crystallization, the product was filtered, washed, dried at 100°C for 12 hours, and calcined at 550°C for 4 hours. A ZSM-23 molecular sieve denoted as Z-2 was obtained.

According to the characterization results of pyridine adsorption FTIR and 2,6-dimethylpyridine adsorption FTIR spectra, the total Brønsted acidity determined via pyridine adsorption FTIR of the molecular sieve Z-2 was 0.179mmol/g, and the total Brønsted acidity determined via 2,6-dimethylpyridine adsorption FTIR was 0.154mmol/g, with their ratio of 86.0: 100. In the Brønsted acidity determined via 2,6-dimethylpyridine adsorption FTIR, the Brønsted acidity at a desorption temperature below 250°C (i.e., the week Brønsted acidity) was 0.130mmol/g. The ratio of the Brønsted acidity at a desorption temperature below 250°C to the total Brønsted acidity determined via 2,6-dimethylpyridine adsorption FTIR was 84.4: 100.

The bulk-phase SiO₂/Al₂O₃ molar ratio of the molecular sieve Z-2 was 246, and the external surface SiO₂/Al₂O₃ molar ratio was 57.

### Example 3

4.68g of hexamethylene diamine was dissolving in 54g of deionized water, followed by the addition of 6g of fumed silica. The mixture was stirred at room temperature (25°C) for 1 hour, and then transferred to a 100mL autoclave equipped with a polytetrafluoroethylene liner. The crystallization reaction was performed at 170°C for 24 hours. After rapid quenching and centrifugal separation, 6.34g of a solid-phase material was obtained.

6mL of oleylamine was mixed with the aforementioned solid-phase material, and the mixture was thoroughly homogenized using an oscillator, followed by stirring at room temperature for 0.5 hour at a stirring speed of 200 rpm. Then 63mL of NaOH solution with a concentration of 0.005mol/L was subsequently added into the mixed liquor, and the primary reaction was carried out at a temperature of 25°C under stirring with a stirring speed of 200rpm for 6 hours to obtain Mixture A. Subsequently, 0.18g of ZSM-23 seeds were dispersed in deionized water to prepare 10mL of ZSM-23 seed suspension, which was then introduced into the Mixture A. The secondary reaction was performed at 80°C under stirring with a stirring speed of 200rpm for 16 hours, yielding Mixture B.

0.17g of Al₂(SO₄)₃·18H₂O and 0.40g of pyrrolidine were added into 20mL of deionized water in sequence to obtain a clear solution C. The solution C was incorporated into the Mixture B and then uniformly blended. The final mixture was transferred into a 150mL autoclave equipped with a polytetrafluoroethylene liner and subjected to crystallization at 200°C for 48 hours. After crystallization, the product was filtered, washed, dried at 100°C for 12 hours, and calcined at 550°C for 4 hours. A ZSM-23 molecular sieve denoted as Z-3 was obtained.

According to the characterization results of pyridine adsorption FTIR and 2,6-dimethylpyridine adsorption FTIR spectra, the total Brønsted acidity determined via pyridine adsorption FTIR of the molecular sieve Z-3 was 0.171mmol/g, and the total Brønsted acidity determined via 2,6-dimethylpyridine adsorption FTIR was 0.131mmol/g, with their ratio of 76.6: 100. In the Brønsted acidity determined via 2,6-dimethylpyridine adsorption FTIR , the Brønsted acidity at a desorption temperature below 250°C (i.e., the week Brønsted acidity) was 0.104mmol/g. The ratio of the Brønsted acidity at a desorption temperature below 250°C to the total Brønsted acidity determined via 2,6-dimethylpyridine adsorption FTIR was 79.4: 100.

The bulk-phase SiO₂/Al₂O₃ molar ratio of the molecular sieve Z-3 was 387, and the external surface SiO₂/Al₂O₃ molar ratio was 71.

### Example 4

4.68g of hexamethylene diamine was dissolving in 54g of deionized water, followed by the addition of 6g of fumed silica. The mixture was stirred at room temperature (25°C) for 1 hour, and then transferred to a 100mL autoclave equipped with a polytetrafluoroethylene liner. The crystallization reaction was performed at 150°C for 24 hours. After rapid quenching and centrifugal separation, 6.61 g of a solid-phase material was obtained.

3.3mL of oleylamine was mixed with the aforementioned solid-phase material, and the mixture was thoroughly homogenized using an oscillator, followed by stirring at room temperature for 0.5 hour at a stirring speed of 250 rpm. Then 30mL of NaOH solution with a concentration of 0.008mol/L was subsequently added into the mixed liquor, and the primary reaction was carried out at 25°C under stirring with a stirring speed of 250rpm for 8 hours to obtain Mixture A. Subsequently, 0.03g of ZSM-23 seeds were dispersed in deionized water to prepare 5mL of ZSM-23 seed suspension, which was then introduced into the Mixture A. The secondary reaction was performed at 60°C under stirring with a stirring speed of 200rpm for 30 hours, yielding Mixture B.

0.44g of Al₂(SO₄)₃·18H₂O and 0.15g of pyrrolidine were added into 15mL of deionized water in sequence to obtain a clear solution C. The solution C was incorporated into the Mixture B and then uniformly blended. The final mixture was transferred into a 100mL reaction autoclave with a polytetrafluoroethylene liner and subjected to crystallization at 180°C for 24 hours. After crystallization, the product was filtered, washed, dried at 100°C for 12 hours, and calcined at 550°C for 4 hours. A ZSM-23 molecular sieve demoted as Z-4 was obtained.

According to the characterization results of pyridine adsorption FTIR and 2,6-dimethylpyridine adsorption FTIR spectra, the total Brønsted acidity determined via pyridine adsorption FTIR of the molecular sieve Z-4 was 0.123mmol/g, and the total Brønsted acidity determined via 2,6-dimethylpyridine adsorption FTIR was 0.104mmol/g, with their ratio of 84.6: 100. In the Brønsted acidity determined via 2,6-dimethylpyridine adsorption FTIR, the Brønsted acidity at a desorption temperature below 250°C (i.e., the week Brønsted acidity) was 0.091mmol/g. The ratio of the Brønsted acidity at a desorption temperature below 250°C to the total Brønsted acidity determined via 2,6-dimethylpyridine adsorption FTIR was 87.5: 100.

The bulk-phase SiO₂/Al₂O₃ molar ratio of the molecular sieve Z-4 was 137, and the external surface SiO₂/Al₂O₃ molar ratio was 64.

### Example 5

The ZSM-23 molecular sieve was prepared according to the method of Example 1, except that the oleylamine was replaced with the same volume of dodecylamine. A ZSM-23 molecular sieve denoted as Z-5 was obtained.

According to the characterization results of pyridine adsorption FTIR and 2,6-dimethylpyridine adsorption FTIR spectra, the total Brønsted acidity determined via pyridine adsorption FTIR of the molecular sieve Z-5 was 0.137mmol/g, and the total Brønsted acidity determined via 2,6-dimethylpyridine adsorption FTIR was 0.109mmol/g, with their ratio of 79.6: 100. In the Brønsted acidity determined via 2,6-dimethylpyridine adsorption FTIR, the Brønsted acidity at a desorption temperature below 250°C (i.e., the week Brønsted acidity) was 0.077mmol/g. The ratio of the Brønsted acidity at a desorption temperature below 250°C to the total Brønsted acidity determined via 2,6-dimethylpyridine adsorption FTIR was 70.6: 100.

The bulk-phase SiO₂/Al₂O₃ molar ratio of the molecular sieve Z-5 was 122, and the external surface SiO₂/Al₂O₃ molar ratio was 81.

### Example 6

The ZSM-23 molecular sieve was prepared according to the method of Example 1, except that the oleylamine was replaced with the same volume of undecylamine. A ZSM-23 molecular sieve denoted as Z-6 was obtained. According to the characterization results of pyridine adsorption FTIR and 2,6-dimethylpyridine adsorption FTIR spectra, the total Brønsted acidity determined via pyridine adsorption FTIR of the molecular sieve Z-6 was 0.118mmol/g, and the total Brønsted acidity determined via 2,6-dimethylpyridine adsorption FTIR was 0.092mmol/g, with their ratio of 77.9: 100. In the Brønsted acidity determined via 2,6-dimethylpyridine adsorption FTIR, the Brønsted acidity at a desorption temperature below 250°C (i.e., the week Brønsted acidity) was 0.063mmol/g. The ratio of the Brønsted acidity at a desorption temperature below 250°C to the total Brønsted acidity determined via 2,6-dimethylpyridine adsorption FTIR was 68.5: 100.

The bulk-phase SiO₂/Al₂O₃ molar ratio of the molecular sieve Z-6 was 133, and the external surface SiO₂/Al₂O₃ molar ratio was 90.

### Example 7

4.68g of hexamethylene diamine was dissolving in 54g of deionized water, followed by the addition of 6g of fumed silica. The mixture was stirred at room temperature (25°C) for 1 hour, and then transferred to a 100mL autoclave equipped with a polytetrafluoroethylene liner. The crystallization reaction was performed at 200°C for 18 hours. After rapid quenching and centrifugal separation, 6.41 g of a solid-phase material was obtained.

12mL of dodecylamine was mixed with the aforementioned solid-phase material, and the mixture was thoroughly homogenized using an oscillator, followed by stirring at room temperature for 0.5 hour at a stirring speed of 200 rpm. Then 40mL of NaOH solution with a concentration of 0.01mol/L was subsequently added into the mixed liquor, and the primary reaction was carried out at 25°C under stirring with a stirring speed of 200rpm for 12 hours to obtain Mixture A. Subsequently, 0.18g of ZSM-23 seeds were dispersed in deionized water to prepare 10mL of ZSM-23 seed suspension, which was then introduced into the Mixture A. The secondary reaction was performed at 80°C under stirring with a stirring speed of 300rpm for 16 hours, yielding Mixture B.

0.17g of Al₂(SO₄)₃·18H₂O and 0.40g of pyrrolidine were added into 20mL of deionized water in sequence to obtain a clear solution C. The solution C was incorporated into the Mixture B and then uniformly blended. The final mixture was transferred into a 150mL autoclave equipped with a polytetrafluoroethylene liner and subjected to crystallization at 180°C for 48 hours. After crystallization, the product was filtered, washed, dried at a 100°C for 12 hours, and calcined at 550°C for 4 hours. A ZSM-23 molecular sieve denoted as Z-7 was obtained.

According to the characterization results of pyridine adsorption FTIR and 2,6-dimethylpyridine adsorption FTIR spectra, the total Brønsted acidity determined via pyridine adsorption FTIR of the molecular sieve Z-7 was 0.180mmol/g, and the total Brønsted acidity determined via 2,6-dimethylpyridine adsorption FTIR was 0.172mmol/g, with their ratio of 95.6: 100. In the Brønsted acidity determined via 2,6-dimethylpyridine adsorption FTIR , the Brønsted acidity at a desorption temperature below 250°C (i.e., the week Brønsted acidity) was 0.159mmol/g. The ratio of the Brønsted acidity at a desorption temperature below 250°C to the total Brønsted acidity determined via 2,6-dimethylpyridine adsorption FTIR was 92.4: 100.

The bulk-phase SiO₂/Al₂O₃ molar ratio of the molecular sieve Z-7 was 364, and the external surface SiO₂/Al₂O₃ molar ratio was 82.

### Example 8

The ZSM-23 molecular sieve was prepared according to the method of Example 1, except that 0.40g of pyrrolidine was replaced with 0.40g of isopropylamine. A ZSM-23 molecular sieve denoted as Z-8 was obtained. According to the characterization results of pyridine adsorption FTIR and 2,6-dimethylpyridine adsorption FTIR spectra, the total Brønsted acidity determined via pyridine adsorption FTIR of the molecular sieve Z-8 was 0.134mmol/g, and the total Brønsted acidity determined via 2,6-dimethylpyridine adsorption FTIR was 0.096mmol/g, with their ratio of 71.6: 100. In the Brønsted acidity determined via 2,6-dimethylpyridine adsorption FTIR, the Brønsted acidity at a desorption temperature below 250°C (i.e., the week Brønsted acidity) was 0.070mmol/g. The ratio of the Brønsted acidity at a desorption temperature below 250°C to the total Brønsted acidity determined via 2,6-dimethylpyridine adsorption FTIR was 72.9: 100.

The bulk-phase SiO₂/Al₂O₃ molar ratio of the molecular sieve Z-8 was 203, and the external surface SiO₂/Al₂O₃ molar ratio was 87.

### Example 9

4.68g of hexamethylene diamine was dissolving in 54g of deionized water, followed by the addition of 6g of fumed silica. The mixture was stirred at room temperature (25°C) for 1 hour, and then transferred to a 100mL autoclave equipped with a polytetrafluoroethylene liner. The crystallization reaction was performed at 150°C for 24 hours. After a rapid quenching and centrifugal separation, 6.43 g of a solid-phase material was obtained.

10mL of oleylamine was mixed with the aforementioned solid-phase material, and the mixture was horoughly homogenized using an oscillator, followed by stirring at room temperature for 0.5 hour at a stirring speed of 200 rpm. Then 42mL of NaOH solution with a concentration of 0.005mol/L was subsequently added into the mixed liquor, and the primary reaction was carried out at a temperature of 25°C under stirring with a stirring speed of 200rpm for 6 hours to obtain Mixture A. Subsequently, 0.18g of ZSM-23 seeds was dispersed in deionized water to prepare 10mL of ZSM-23 seed suspension, which was then introduced into the Mixture A. The secondary reaction was performed at 80°C under stirring with a stirring speed of 300rpm for 16 hours, yielding Mixture B.

0.34g of Al₂(SO₄)₃·18H₂O and 0.35g of isopropylamine were added into 20mL of deionized water in sequence to obtain a clear solution C. The solution C was incorporated into the Mixture B and then uniformly blended. The final mixture was transferred into a 150mL autoclave equipped with a polytetrafluoroethylene liner and subjected to crystallization at 170°C for 48 hours. After crystallization, the product was filtered, washed, dried at 100°C for 12 hours, and calcined at 550°C for 4 hours. A ZSM-23 molecular sieve denoted as Z-9 was obtained.

According to the characterization results of pyridine adsorption FTIR and 2,6-dimethylpyridine adsorption FTIR spectra, the total Brønsted acidity determined via pyridine adsorption FTIR of the molecular sieve Z-9 was 0.124mmol/g, and the total Brønsted acidity determined via 2,6-dimethylpyridine adsorption FTIR was 0.103mmol/g, with their ratio of 83.0: 100. In the Brønsted acidity determined via 2,6-dimethylpyridine adsorption FTIR, the Brønsted acidity at a desorption temperature below 250°C (i.e., the week Brønsted acidity) was 0.074mmol/g. The ratio of the Brønsted acidity at a desorption temperature below 250°C to the total Brønsted acidity determined via 2,6-dimethylpyridine adsorption FTIR was 71.8: 100.

The bulk-phase SiO₂/Al₂O₃ molar ratio of the molecular sieve Z-9 was 189, and the external surface SiO₂/Al₂O₃ molar ratio was 88.

### Example 10

4.68g of hexamethylene diamine was dissolving in 54g of deionized water, followed by the addition of 6g of fumed silica. The mixture was stirred at room temperature (25°C) for 1 hour, and then transferred to a 100mL autoclave equipped with a polytetrafluoroethylene liner. The crystallization reaction was performed at a temperature of 200°C for 18 hours. After rapid quenching and centrifugal separation, 6.41 g of a solid-phase material was obtained.

12mL of oleylamine was mixed with the aforementioned solid-phase material, and the mixture was thoroughly homogenized using an oscillator, followed by stirring at room temperature for 0.5 hour at a stirring speed of 200 rpm. Then 42mL of NaOH solution with a concentration of 0.01mol/L was subsequently added into the mixed liquor, and the primary reaction was carried out at 25°C under stirring with a stirring speed of 200rpm for 12 hours to obtain Mixture A. Subsequently, 0.18g of ZSM-23 seeds were dispersed in deionized water to prepare 10mL of ZSM-23 seed suspension, which was then introduced into the Mixture A. The secondary reaction was performed at 80°C under stirring with a stirring speed of 300rpm for 16 hours, yielding Mixture B.

0.17g of Al₂(SO₄)₃·18H₂O and 0.40g of isopropylamine were added into 20mL of deionized water in sequence to obtain a clear solution C.The solution C was incorporated into the Mixture B and then uniformly blended. The final mixture was transferred into a 150mL autoclave equipped with a polytetrafluoroethylene liner and subjected to crystallization at 180°C for 48 hours. After crystallization, the product was filtered, washed, dried at 100°C for 12 hours, and calcined at 550°C for 4 hours. A ZSM-23 molecular sieve denoted as Z-10 was obtained.

According to the characterization results of pyridine adsorption FTIR and 2,6-dimethylpyridine adsorption FTIR spectra, the total Brønsted acidity determined via pyridine adsorption FTIR of the molecular sieve Z-10 was 0.127mmol/g, and the total Brønsted acidity determined via 2,6-dimethylpyridine adsorption FTIR was 0.114mmol/g, with their ratio of 89.8: 100. In the Brønsted acidity determined via 2,6-dimethylpyridine adsorption FTIR, the Brønsted acidity at a desorption temperature below 250°C (i.e., the week Brønsted acidity) was 0.103mmol/g. The ratio of the Brønsted acidity at a desorption temperature below 250°C to the total Brønsted acidity determined via 2,6-dimethylpyridine adsorption FTIR was 90.3: 100.

The bulk-phase SiO₂/Al₂O₃ molar ratio of the molecular sieve Z-10 was 372, and the external surface SiO₂/Al₂O₃ molar ratio was 75.

### Comparative Example 1

The comparative example was operated with reference to the preparation method of ZSM-23 molecular sieve in the patent application CN109516471A.

0.079g of NaOH, 0.163g of Al₂(SO₄)₃·18H₂O, 0.438g of pyrrolidine(PY) and 1.48g of fumed silica were dissolved in 20.0g of deionized water in sequence. The materials were fully and uniformly mixed to obtain Gel A, wherein the molar ratio of the components was follows: the ratio of SiO₂ in the silicon source : Al₂O₃ in the aluminum source : PY : NaOH : H₂O = 1: 0.01: 0.25: 0.083: 45. The Gel A was placed in a constant-temperature oven for heating at 180°C for 12 hours, and then cooled to room temperature. Meanwhile, 0.119g of NaOH, 0.489g of Al₂(SO₄)₃·18H₂O and 2.22g of fumed silica were sequentially dissolved in 30.0g of deionized water, and the materials were uniformly stirred to obtain Gel B. The molar ratio of the components was as follows: the ratio of SiO₂ in the silicon source : Al₂O₃ in the aluminum source : NaOH : H₂O = 1: 0.02: 0.083: 45. A uniform white gel was obtained after the Gel B was added into the Gel A under stirring for 1 hour. The mixture was then transferred into a 100mL autocalve and subjected to crystallization at 180°C for 44 hours. The obtained product was subjected to filtering, washing, and drying at 65°C for 24 hours, and A ZSM-23 molecular sieve denoted as DZ-1 was obtained. According to the characterization results of pyridine adsorption FTIR and 2,6-dimethylpyridine adsorption FTIR spectra, the total Brønsted acidity determined via pyridine adsorption FTIR of the molecular sieve DZ-1 was 0.271mmol/g, and the total Brønsted acidity determined via 2,6-dimethylpyridine adsorption FTIR was 0.116mmol/g, with their ratio of 42.8: 100. In the Brønsted acidity determined via 2,6-dimethylpyridine adsorption FTIR, the Brønsted acidity at a desorption temperature below 250°C (i.e., the week Brønsted acidity) was 0.034mmol/g. The ratio of the Brønsted acidity at a desorption temperature below 250°C to the total Brønsted acidity determined via 2,6-dimethylpyridine adsorption FTIR was 29.3: 100.

The bulk-phase SiO₂/Al₂O₃ molar ratio of the molecular sieve DZ-1 was 48, and the external surface SiO₂/Al₂O₃ molar ratio was 44.

### Comparative Example 2

The ZSM-23 molecular sieve was prepared according to the method of Example 1, except that oleylamine was not added. A ZSM-23 molecular sieve denoted as DZ-2 was obtained.

According to the characterization results of pyridine adsorption FTIR and 2,6-dimethylpyridine adsorption FTIR spectra, the total Brønsted acidity determined via pyridine adsorption FTIR of the molecular sieve DZ-2 was 0.176mmol/g, and the total Brønsted acidity determined via 2,6-dimethylpyridine adsorption FTIR was 0.081mmol/g, with their ratio of 46.0: 100. In the Brønsted acidity determined via 2,6-dimethylpyridine adsorption FTIR, the Brønsted acidity at a desorption temperature below 250°C (i.e., the week Brønsted acidity) was 0.037mmol/g. The ratio of the Brønsted acidity at a desorption temperature below 250°C to the total Brønsted acidity determined via 2,6-dimethylpyridine adsorption FTIR was 45.7: 100.

The bulk-phase SiO₂/Al₂O₃ molar ratio of the molecular sieve DZ-2 was 94, and the external surface SiO₂/Al₂O₃ molar ratio was 89.

The parameters associated with the ZSM-23 molecular sieves prepared in the aforementioned Examples and Comparative Examples were shown in Table 1.

**Table 1**

| | Ratio of total Brønsted acidity determined via 2,6-dimethylpyridine adsorption FTIR to total Brønsted acidity determined via pyridine adsorption FTIR | Ratio of Brønsted acidity at a desorption temperature below 250°C to total Brønsted acidity determined via 2,6-dimethylpyridine adsorption FTIR | bulk-phase SiO₂/ Al₂O₃ | external surface SiO₂/ Al₂O₃ |
|---|---|---|---|---|
| Example 1 | 89.1: 100 | 90.8: 100 | 97 | 46 |
| Example 2 | 86.0: 100 | 84.4: 100 | 246 | 57 |
| Example 3 | 76.6: 100 | 79.4: 100 | 387 | 71 |
| Example 4 | 84.6: 100 | 87.5: 100 | 137 | 64 |
| Example 5 | 79.6: 100 | 70.6: 100 | 122 | 81 |
| Example 6 | 77.9: 100 | 68.5: 100 | 133 | 90 |
| Example 7 | 95.6: 100 | 92.4: 100 | 364 | 82 |
| Example 8 | 71.6: 100 | 72.9: 100 | 203 | 87 |
| Example 9 | 83.0: 100 | 71.8: 100 | 189 | 88 |
| Example 10 | 89.8: 100 | 90.3: 100 | 372 | 75 |
| Comparative Example 1 | 42.8: 100 | 29.3: 100 | 48 | 44 |
| Comparative Example 2 | 46.0: 100 | 45.7: 100 | 94 | 89 |

### Application Examples 1-10 and Application Comparative Examples 1-2

The ZSM-23 molecular sieve samples prepared in Examples 1-10 and Comparative Examples 1-2 were used as supports, respectively. 0.5wt.% noble metal Pt was loaded on the supports through an excessimpregnation method to obtain catalyst powders.

The properties of the ZSM-23 molecular sieve samples were evaluated on a fixed bed micro-reactor. The catalyst powders were tableted, crushed into particles of 5-10 meshes, and loaded into a fixed-bed microreactor. Ethyl cyclohexane hydrogenation reaction was selected as the model reaction. The reaction raw material was a mixture composed of 90wt.% of decalin and 10wt.% of ethyl cyclohexane. The reaction conditions were set as follows: reaction temperature 280°C, liquid hourly space velocity 1.0h⁻¹, hydrogen-oil volume ratio 800, and hydrogen pressure 4.0 MPa. The catalytic results were listed in Table 2.

**Table 2**

| | Conversion rate of ethyl cyclohexane, wt% | C₈H₁₈ yield, wt% | C₈H₁₈ isomerization degree, % | Ratio of multiple branched chain component to the single branched chain component in C₈H₁₈ isomerization product |
|---|---|---|---|---|
| Example 1 | 93.2 | 78.1 | 98.0 | 2.4 |
| Example 2 | 94.1 | 77.6 | 97.5 | 2.7 |
| Example 3 | 93.5 | 77.8 | 98.2 | 2.3 |
| Example 4 | 92.6 | 78.9 | 97.3 | 2.8 |
| Example 5 | 92.7 | 77.9 | 96.8 | 2.1 |
| Example 6 | 93.3 | 80.2 | 98.1 | 1.8 |
| Example 7 | 92.4 | 75.7 | 94.7 | 2.5 |
| Example 8 | 93.0 | 79.5 | 95.6 | 1.6 |
| Example 9 | 93.2 | 78.4 | 97.4 | 1.7 |
| Example 10 | 92.3 | 74.8 | 92.1 | 2.0 |
| Comparative Example 1 | 93.6 | 34.9 | 68.4 | 0.5 |
| Comparative Example 2 | 92.9 | 41.6 | 71.0 | 0.7 |

Seen from the results in Table 2, the hydrogenation catalysts supported on the ZSM-23 molecular sieve according to the present invention exhibit significantly superior performance in the hydrogenation ring-opening and hydroisomerization of cycloparaffins.

The above content describes in detail the preferred embodiments of the present invention, but the present invention is not limited thereto. A variety of simple modifications can be made in regard to the technical solutions of the present invention within the scope of the technical concept of the present invention, including a combination of individual technical features in any other suitable manner. Such simple modifications and combinations thereof shall also be regarded as the content disclosed by the present invention, and each of them falls into the protection scope of the present invention.

## Claims

1. A ZSM-23 molecular sieve, is **characterized in that** the ZSM-23 molecular sieve possesses a ratio of total Brønsted acidity determined via 2,6-dimethylpyridine adsorption FTIR to total Brønsted acidity determined via pyridine adsorption FTIR being (65-99): 100, preferably (70-97): 100, and more preferably (74-95): 100; in the Brønsted acidity determined via 2,6-dimethylpyridine adsorption FTIR, the ratio of Brønsted acidity at a desorption temperature below 250°C to the total Brønsted acidity determined via 2,6-dimethylpyridine adsorption FTIR is (64-95): 100, preferably (67-93):100, and more preferably (70-91): 100.

2. The ZSM-23 molecular sieve according to claim 1, wherein the total Brønsted acidity determined via pyridine adsorption FTIR of the ZSM-23 molecular sieve is within the range of 0.1-0.37mmol/g, preferably within the range of 0.13-0.35mmol/g, and more preferably within the range of 0.15-0.33mmol/g.

3. The ZSM-23 molecular sieve according to claim 1 or 2,wherein the total Brønsted acidity determined via 2,6-dimethylpyridine adsorption FTIR of the ZSM-23 molecular sieve is within the range of 0.07-0.35mmol/g, preferably within the range of 0.09-0.33mmol/g, and more preferably within the range of 0.11-0.30mmol/g.

4. The ZSM-23 molecular sieve according to any one of claims 1-3, wherein in the Brønsted acidity determined via 2,6-dimethylpyridine adsorption FTIR of the ZSM-23 molecular sieve, the Brønsted acidity at a desorption temperature below 250°C is within the range of 0.06-0.31mmol/g, preferably within the range of 0.07-0.30mmol/g, and more preferably within the range of 0.1-0.26mmol/g.

5. The ZSM-23 molecular sieve according to any one of claims 1-4, wherein the ZSM-23 molecular sieve possesses a bulk-phase SiO₂/Al₂O₃ molar ratio higher than an external surface SiO₂/Al₂O₃ molar ratio by an amount of 40-400, preferably an amount of 45-350.

6. The ZSM-23 molecular sieve according to claim 1 or 5, wherein the ZSM-23 molecular sieve possesses a bulk-phase SiO₂/Al₂O₃ molar ratio within the range of 70-500 and an external surface SiO₂/Al₂O₃ molar ratio within the range of 40-120;
Preferably, the ZSM-23 molecular sieve possesses a bulk-phase SiO₂/Al₂O₃ within the range of 90-400 and an external surface SiO₂/Al₂O₃ molar ratio within the range of 45-90.

7. A method for preparing ZSM-23 molecular sieve is **characterized in that** the method comprises the following steps:
(1) Crystallizing a mixture of silicon source, template agent a and water, and then separating the solid-phase material from the resultant product;
(2) Mixing the above solid-phase material with aliphatic amine and inorganic base for a primary reaction, subsequently adding ZSM-23 seeds and proceeding with a secondary reaction;
(3) Blending the mixed material obtained in step (2) with an aluminum source and a template agent b to obtain a mixture, followed by crystallization, filtration, washing, drying and calcination in sequence.

8. The method according to claim 7, wherein the molar ratio of the dosage of water, said template a, and said silicon source calculated in terms of SiO₂ in step (1) is (20-80): (0.1-1): 1, preferably (30-70): (0.15-0.8): 1; Preferably, the silicon source is one or a random combination of at least two of white carbon black, silica sol, sodium silicate, fumed silica and tetraethyl orthosilicate;
Preferably, the template agent a is one or a random combination of at least two of hexamethylene diamine, n-hexylamine, ethanol, tetrapropyl ammonium hydroxide, tetrapropyl ammonium bromide and triethylamine; Preferably, the crystallization reaction conditions comprise a temperature within the range of 120-220°C and a duration of 8-48 hours.

9. The method according to claim 7 or 8, wherein the liquid-solid ratio of the aliphatic amine to the solid-phase material in step (2) is within the range of 0.3-3 mL/g, preferably within the range of 0.5-2 mL/g;
Preferably, the aliphatic amine is C₁₂-C₁₈ aliphatic amine, more preferably at least one selected from the group consisting of oleylamine, octadecylamine and dodecylamine.

10. The method according to any one of claims 7-9, wherein the inorganic base in step (2) is used in the form of an alkali solution, and the concentration of the alkali solution is within the range of 0.003-0.015mol/L, preferably within the range of 0.005-0.01 mol/L;
Preferably, the liquid-solid ratio of the alkali solution to the solid-phase material is within the range of 2-15 mL/g, more preferably within the range of 4-10 mL/g;
Preferably, the inorganic base is at least one selected from the group of sodium hydroxide, potassium hydroxide and ammonia water.

11. The method according to any one of claims 7-10, wherein the temperature of the secondary reaction in step (2) is higher than that of the primary reaction;
Preferably, the conditions of the primary reaction comprise: a temperature within the range of 20-40°C, and a duration of 3-15 hours;
Preferably, the conditions of the secondary reaction comprise: a temperature within the range of 60-120°C, and a duration of 6-30 hours.

12. The method according to any one of claims 7-11, wherein the dosage of said ZSM-23 seeds in step (2) is 0.1-8 parts by weight, preferably 0.5-5 parts by weight, relative to 100 parts by weight of the solid-phase material.

13. The method according to any one of claims 7-12, wherein the molar ratio of the dosage of said template b, the aluminum source calculated in terms of Al₂O₃, and the mixed material calculated in terms of SiO₂ in step (3) is (0.01-0.1): (0.002-0.015): 1, preferably (0.02-0.08): (0.005-0.01): 1; Preferably, the template agent b is one or a random combination of at least two of pyrrolidine, isopropylamine, N,N-dimethylformamide, dimethylamine and ethylenediamine;
Preferably, the aluminum source is one or a random combination of at least two of aluminum sulfate, aluminum isopropoxide, sodium aluminate and aluminum hydroxide.

14. The method according to any one of claims 7-13, wherein the crystallizing conditions in step (3) comprisea temperature within the range of 180-220°C, and a duration of 24-72 hours;
Preferably, the drying conditions comprise: a temperature within the range of 80-120°C, and a duration of 6-12 hours;
Preferably, the calcining conditions comprise: a temperature within the range of 540-560°C, and a duration of 3-8 hours.

15. A ZSM-23 molecular sieve prepared with the method according to any one of claims 7-14.

16. A hydrogenation catalyst comprising a support and an active component attached on the support, is **characterized in that** the support is the ZSM-23 molecular sieve according to any one of claims 1-6 and 15.

17. Use of the hydrogenation catalyst according to claim 16 in the long-chain alkane hydrocracking reactions, olefin hydrocracking reactions or alkane isomerization reactions.
